(19) Europäisches Patentamt
European Patent Office
Office européen des brevets

(11) EP 1 927 363 B1

# (12) EUROPEAN PATENT SPECIFICATION

(45) Date of publication and mention of the grant of the patent:
**26.02.2014 Bulletin 2014/09**

(21) Application number: **07764124.9**

(22) Date of filing: **26.06.2007**

(51) Int Cl.:
***A61K 35/64*** *(2006.01)* ***A61P 7/02*** *(2006.01)*
***A61K 35/62*** *(2006.01)*

(86) International application number:
**PCT/CN2007/070190**

(87) International publication number:
**WO 2008/003259 (10.01.2008 Gazette 2008/02)**

(54) **AN EXTRACT FOR PREVENTING OR TREATING THROMBOTIC DISEASES**

EXTRAKT ZUR VORBEUGUNG UND BEHANDLUNG VON THROMBOSEN

EXTRAIT DESTINÉ À PRÉVENIR OU À TRAITER DES TROUBLES THROMBOTIQUES

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **28.06.2006 CN 200610086680**

(43) Date of publication of application:
**04.06.2008 Bulletin 2008/23**

(73) Proprietor: **Mudanjiang Youbo Pharmaceutical Co. Ltd**
**Aimin District**
**Mudanjiang City**
**Heilongjiang 157011 (CN)**

(72) Inventor: **Li, Zhenguo**
**Mudanjiang City**
**Heilongjiang 157011 (CN)**

(74) Representative: **Petho, Arpad et al**
**Danubia**
**Patent & Law Office LLC**
**Bajcsy-Zsilinszky út 16**
**1051 Budapest (HU)**

(56) References cited:
**WO-A1-97/17981 CN-A- 1 651 079**
**CN-A- 1 660 145 CN-C- 1 192 782**
**US-A- 4 767 742 US-A- 5 246 715**
**US-A- 5 861 377**

- **SEYMOUR J L ET AL: "DECORSIN A POTENT GLYCOPROTEIN IIB-IIIA ANTAGONIST AND PLATELET AGGREGATION INHIBITOR FROM THE LEECH MACROBDELLA-DECORA", JOURNAL OF BIOLOGICAL CHEMISTRY, vol. 265, no. 17, 1990, pages 10143-10147, XP009158019, ISSN: 0021-9258**
- **YANES OSCAR ET AL: "Functional screening of serine protease inhibitors in the medical leech Hirudo medicinalis monitored by intensity fading MALDI-TOF MS", MOLECULAR & CELLULAR PROTEOMICS, vol. 4, no. 10, October 2005 (2005-10), pages 1602-1613, XP002672736, ISSN: 1535-9476**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 927 363 B1

## Description

### Field of Invention

The present invention relates to an extract for use in preventing or treating thrombotic diseases, and more particularly to an extract of leech and earthworm with molecular weight of less than 5,800 Dalton, pharmaceutical compositions comprising and processes for preparation and use thereof.

### Description of Related Arts

As a kind of classic drug of removing blood clot, the Chinese Pharmacopeia records three kinds of leech-Whitmania pigra Whitman, Hirudo nipponica Whitman and Whitmania ac-ranulata Whitman. Among them, Whitmania pigra Whitman is the most widely-used for drug. The main ingredients of leech are protein, polypeptide, amino acids, nucleic acid, enzyme, sugars, trace elements and etc. The qualitative and quantitative researches about ingredients in leech such as protein, amino acids, sugars, trace elements and etc have been reported for many times. The main methods are TLC, HPLC, and electrophoresis method. The main micromolecular ingredients in leech are hypoxanthine, xanthine, uracil, uracil riboside and etc. Both Pyrimidine and Purine are unsaturated heterocyclic compounds containing Nitrogen atoms.

As Chinese traditional medicine, earthworm in the Chinese Pharmacopeia has effects of removing heat, calming, getting through arteries and veins, smoothening asthma and diuresis. There are four kinds of earthworm in the Chinese pharmacopeia. They are Pheretima aspergillum (E.Perrier), Pheretima vulgaris Chen, Pheretima guillelmi (Michaelsen) and Pheretima pectinifera Michaelsen. Among them, Pheretima aspergillum (E.Perrier) also known as Lumbricus is the most widely-used for drug. The main ingredients in earthworm are protein, amino acids, nucleic acid, enzyme, sugars and etc. The qualitative and quantitative researches about ingredients in earthworm have been reported. The main micromolecular ingredients in earthworm are hypoxanthine and compounds containing Nitrogen atoms.

Extract of leech and earthworm is the active components in Shuxuetong Injection which possesses high performance and immediate effect in treating cardiovascular and cerebrovascular diseases. Chinese patent applications 03148281.3, 200410101538.8 and 200510000266.7 have disclosed Shuxuetong Injection and process for preparation thereof respectively. All content of these applications were mentioned in the present invention as reference.

Materials possessing immunogenicity usual are macromolecule, and the molecular weight is larger, the immunogenicity is stronger. Micromolecule polypeptides usually have no or few immunogenicity. Antigens usual are macromolecule because the following probable reasons: (1) The relative molecular weight of antigens are bigger, the superficial specific chemical genes(antigen determinant) are more, so that the effect of stimulating immunologic cells is better and the immunologic response is emerged; (2) Macromolecular antigens have complex chemical compositions and stable structures and can not be destroyed and eliminated easily and can stay in human being's body for long time. So they can stimulate immunologic system persistently and emerge immunologic response. Insulin human composed of 51 amino acids (standard molecular weight of Insulin human is 5800 Dalton) is the boundary of high molecular weight materials and low molecular weight materials.All materials with molecular weight of more than 5,800 Dalton are called high molecular weight materials.The high molecular weight materials are controlled strictly in injection.

US 5,246,715 discloses a composition of blood platelet aggregation comprising an isolated fraction derived from *Hirudinidae* leeches' saliva. US 5,246,715, however, fails to disclose any extract made from both leeches and earthworms.

US 5,861,377 discloses the purification of hirustatin from the medical leech *Hirudo medicinalis* by extracting homogenized leeches with acetone, followed by further purification steps. US 5,861,377, however, does not disclose any extract made from both leeches and earthworms.

WO 97/17981 discloses a compound having antiviral activity, obtainable from leeches by solvent extraction. WO 97/17981, however, does not disclose any extract made from both leeches and earthworms.

Seymour JL et al. (J. Biol. Chem. vol. 265, no. 17, 1990, p.: 10143-10147) disclose the discovery, purification and characterization of decrosin, a protein isolated from the leech species *Macrobdella decora,* which is a potent inhibitor of platelet aggregation. Seymour JL et al., however, do not disclose any extract made from both leeches and earthworms.

Yannes Oscar et al. (Mol. and Cell. Proteom., vol. 4, no. 10, 2005, p.: 1602-1613) disclose functional screening of serine protease inhibitors in the extract of the medical leech *Hirudo medicinalis.* Yannes Oscar et al., however, do not disclose any extract made from both leeches and earthworms.

CN 1 192 782 C, CN 1 651 079 A and CN 1 660 145 A all disclose different extracts made of both leeches and earthworms useful in inhibiting blood clotting but the molecular weight of the disclosed extracts are substantially higher than 5,800 Daltons, which is the preferred molecular weight of the extracts according to the present invention.

Summary of the Present Invention

A main object of the present invention is to provide an extract for preventing or treating thrombotic diseases, and more particularly an extract of leech and earthworm with molecular weight of less than 5,800 Dalton, and process for preparation and use thereof. Comparing with present technology, the extract is safer and more unsuspecting in clinic.

Accordingly, in order to accomplish the above object, the technology programme of present invention is described as follows:

An extract for preventing or treating thrombotic diseases which is extracted from the raw material of leech and earthworm is characterized by molecular weight of less than 5,800 Dalton.

The extract is an extract of leech and earthworm. The extract mixture of leech and earthworm is the mixture of extract of leech and extract of earthworm, or extract of mixture of leech and earthworm.

The leech is *Whitmania pigra Whitman, Hirudo nipponica Whitman* or *Whitmania ac-ranulata Whitman.* The earthworm is *Pheretima aspergillum (E.Perrier), Pheretima vulgaris Chen, Pheretima guillelmi (Michaelsen)* or *Pheretima pectinifera Michaelsen.* In them, dry or fresh *Whitmania pigra Whitman* and *Pheretima uspergillum (E.Perrier)* are preferred.

The extract is obtained by filtration with ultrafiltration membrane or ultrafiltration column which can intercept materials with molecular weight of more than 6,000 Dalton (6,000 Dalton is preferred). For example, the extract (semi-finished products or intermediates) which is obtained by common method of the prior art from the raw material (leech and earthworm) is filtered by the ultrafiltration membrane or ultrafiltration column which can intercept materials with molecular weight of more than 6,000 Dalton (6,000 Dalton is preferred), then the extract of leech and/or earthworm with molecular weight of less than 5,800 Dalton is obtained.

As preference, the extract of the present invention is obtained by the process containing these following steps:

(1) Raw material is cleaned by water for injection or normal saline, steeped at low temperature and filtered. Filtrate and dregs are gained respectively;

(2) Dregs are smashed and mixed with filtrate which is obtained in step (1), and homogenate is obtained. The homogenate is frozen and melted repeatedly, then centrifuged, and supernate is obtained;

(3) Supernate which is obtained in step (2) is ultrafiltrated with ultrafiltration membrane or ultrafiltration column which can intercept materials with molecular weight of between 10,000-100,000 Dalton, and filtrate is obtained;

(4) The filtrate which is obtained in step (3) is ultrafiltrated with ultrafiltration membrane or ultrafiltration column which can intercept materials with molecular weight of more than 6,000 Dalton (6,000Dalton is preferred).

If it is necessary, the filtration process with ultrafiltration membrane or ultrafiltration column which can intercept materials with molecular weight of more than 6,000 Dalton (6,000 Dalton is preferred) is repeated. Thermocompression process is handled before filtration if it is necessary, for instance, filtrate is thermocompressed at 105-136°C for 10-45 minutes.

As preference, the extract of the present invention is obtained by the process containing these following steps:

(1) Raw material is cleaned by water for injection or normal saline and smashed, then homogenate is obtained. The homogenate is frozen and melted repeatedly, then centrifuged, and supernate is obtained;

(2) The supernate which is obtained in step (1) is ultrafiltrated with ultrafiltration membrane or ultrafiltration column which can intercept materials with molecular weight of between 10,000 -100,000 Dalton, and filtrate is obtained;

(3) The filtrate which is obtained in step (2) is handled with method of ion-exchange chromatography;

(4) The eluate which is obtained in step (3) is ultrafiltrated with ultrafiltration membrane or ultrafiltration column which can intercept materials with molecular weight of more than 6000 Dalton after/before the process of desalination.

If it is necessary, filtration process with ultrafiltration membrane or ultrafiltration column which can intercept materials with molecular weight of more than 6,000 Dalton (6,000Dalton is preferred) is repeated.

Ultrafiltration membrane or ultrafiltration column is the common kind in this technology field. Such as hollow fiber ultrafiltration column of which material is polysulfone and cuttting molecular weight is not more than 6,000 Dalton (6,000 Dalton is preferred). Such as hollow fiber ultrafiltration column of which cuttting molecular weight is between 10,000 and 100,000 Dalton (10,000 or 50,000 is preferred).

The ion exchange column is cation exchange column or anion exchange column. Cation exchange column is one of CM-sephadex, CM-agarose, CM-cellulose, SP-sephadex, SP-agarose, SP-cellulose and etc. Anion exchange column is one of DEAE-sephadex, DEAE-agarose, DEAE-cellulose, Q-sephadex, Q-agarose, Q-cellulose and etc.

There are polypeptide, amino acid and hypoxanthine in the extract of the present invention.

Raw material of leech and earthworm is dry or fresh. Raw material of leech and earthworm are extracted respectively, and extract of leech and extract of earthworm are obtained respectively. Or mixture of raw material of leech and earthworm is extracted, and mixture of extract of leech and earthworm is obtained.

The extract of the present invention is liquid or solid after dehydration and drying.

Another object of the present invention is to provide a process for preparation of the extract. The process includes filtration with ultrafiltration membrane or ultrafiltration column which can intercept materials with molecular weight of more than 6,000 Dalton (6,000 Dalton is preferred). For example, raw material of leech and earthworm is extracted and separated with common method of present technology, obtained extract (also is called half-finished product or intermediates) is filtered with ultrafiltration membrane or ultrafiltration column which can intercept materials with molecular weight of more than 6,000 Dalton (6,000 Dalton is preferred). Extract of leech and/or earthworm with molecular weight of less than 5,800 Dalton is obtained.

The following steps of process for preparation of the extract of the present invention are preferred, including:

(1) Raw material is cleaned by water for injection or normal saline, steeped at low temperature and filtered. Filtrate and dregs are gained respectively;

(2) Dregs are smashed and mixed with filtrate which is obtained in step (1), and homogenate is obtained. The homogenate is frozen and melted repeatedly, then centrifuged, and supernate is obtained;

(3) Supernate which is obtained in step (2) is ultrafiltrated with ultrafiltration membrane or ultrafiltration column which can intercept materials with molecular weight of between 10,000 -100,000 Dalton, and filtrate is obtained;

(4) The filtrate which is obtained in step (3) is ultrafiltrated with ultrafiltration membrane or ultrafiltration column which can intercept materials with molecular weight of more than 6,000 Dalton (6,000Dalton is preferred).

If it is necessary, the filtration process with ultrafiltration membrane or ultrafiltration column which can intercept materials with molecular weight of more than 6,000 Dalton (6,000 Dalton is preferred) is repeated. Thermocompression process is handled before filtration if it is necessary, for instance, filtrate is thermocompressed at 105-136°C for 10-45 minutes.

Insulin human of which molecular weight is 5,800 Dalton is reference, and materials of which molecular weight is over the insulin human are high molecular weight materials.

For example, the contract of the present inventiong is determined by HPLC as follow:

A. Materials and instruments

1. Material of standard protein molecular weight: insulin human, molecular weight is 5,800 Dalton, 1ml/ramus, purchased from the National Institute for the Control of Pharmaceutical and Biological Products.

2. Instruments
Visible ultraviolet spectrophotometer (Japanese Hitachi U-3210)
High performance liquid chromatograph Waters 600
High performance liquid chromatograph Agilent Hp1100

3. Reagents
Trifluoroacetic acid, chemical pure (Science and Technology University Chemical Industry and Material Institute)
Acetonitrile, chromatographic pure (Dikma Company)

B. Chromatographic conditions

Chromatographic column: gel chromatographic column (TSK GEL 2000 SWx1 7.8 mm * 300 mm);

Mobile phase: trifluoroacetic acid-acetonitrile-water, their volume ratio is 0.025:30:70;

Ultraviolet detector: detection wave length is 214 ± 1 nm;

Flow rate is 0.7 ml/min.

C. Determination

Suitable quantity sample of extract and insulin human are taken accurately to be mixed with mobile phase respectively, sample solution and control solution are obtained; Sample solution and control solution are taken accurately to be injected into High performance liquid chromatograph respectively, determined with the method of High performance liquid chromatography. Chromatogram is recorded. Content of materials with molecular weight of more than insulin human is determined by calculating content of materials corresponding to chromatographic peak of which retention time is shorter than retention time of insulin human peak. Content of high molecular weight in sample is content of materials with molecular weight of more than 5,800 Dalton in sample.

Distribution of molecular weight of extract is determined with MS. For example, Distribution of molecular weight of extract is determined with the method as follow:

Suitable quantity sample of extract (or desalinatd if necessary) is dissolved in 0.5%TFA solution. Sample solution and matrix are mixed together with ratio of 1:1, targeted and dried at room temperature. The of extract is DHB (2, 5-dihydroxy benzoic acid) or CCA (a-cyano-4-hydroxy cinnamic acid).

Determination instrument is Autoflex (Bruker Company). MS condition (MALDI-TOF-MS); N2 laser source of which wave length is 337nm; linear scanning (length of flying pipe is 1.6m, accelerating voltage is 20kv); Ion type is positive ion or negative ion.

Determination result shows that the molecular weight of all components of extract of the present invention is less than 5,800 Dalton.

In other words, after being determined with the method above, there isn't material with molecular weight of more than 5,800 Dalton. So molecular weight of the extract for preventing or treating thrombotic diseases is not more than 5,800 Dalton.

There are polypeptide, amino acids and hypoxanthine in the extract. Polypeptide and amino acids are analyzed with suitable amino acid analyzer or High performance liquid chromatograph. For example, method of HPLC in Chinese pharmacopeia 2005 edition 1 st section appendix VI D is referred to determine content of amino acid:

Chromatographic condition and systematic adaptivity test: filler is octadecyl silane bonded silica gel; mobile phase is A liquid or B liquid, gradient elution, detection wave length is 338 nm and 262 nm, column temperature is 30-40°C.

A liquid: suitable quantity sodium acetate is dissolved in water for injection, and then triethylamine is added. Mix well, PH is adjusted to neutral, and then THF is added.

B liquid: suitable quantity sodium acetate is dissolved in water for injection, and then PH is adjusted to neutral with acetic acid. Suitable quantity Mixed liquid of acetonitrile and methanol (1:1) is added, mix well.

Suitable quantity boric acid buffer solution of PH 10.4 is added in suitable quantity control of amino acid, and o-phthalaldehyde and 9- fluorene methyl chlorine formate derivative reagent are added. The mixture is stirred uniformly in order to react sufficiently and get o-phthalic acid-9- fluorene methyl chlorine formate amino acid which is control solution. As sample, extract of the present invention is handled with the same method, and amino acid in extract is determined with high performance liquid chromatograph.

Method of HPLC in Chinese pharmacopeia 2005 edition 1th section appendix VI D is referred to determine content of hypoxanthine. Fixed phase is octadecyl silanebonded silica gel, mobile phase is 0.1% dibasic sodium phosphate solution, and detection wave length is 254nm. Theoretical plate number is not less than 3000 according to hypoxanthine.

In local stimulative test of rabbit, 1 ml the extract solution of which concentration is 0.5 g/ml is injected into left and right legs musculus quadriceps of rabbit. Rabbit is put to death 48 hours later. Both leg quadriceps of rabbit is taken out and incised longitudinal. After observing local stimulative reaction, the musculus quadriceps is put into 10% formaldehyde solution and checked up with common method of histopathology. Result: there is not congestion and necrosis in leg musculus quadriceps of rabbit with naked eyes. The value is 0 according to reaction grade in stimulative reaction, in other words, there is not evident change. It is confirmed that there isn't evident inflammatory reaction through pathology inspection. It also shows that the extract can't cause evident local simulative reaction.

In blood vessel simulative test of rabbit, 1 g/kg (25 times of quantity in clinic) the extract solution of which concentration is 0.5 g/ml is injected into ear vein of rabbit 1 time/day for 3 days. Pieces of ear of injection position is fixed and checked up with common method of histopathology. To observe if there is inflammatory reaction in injected vein. Result: it is showed that there isn't denatured and necrotic tissue and repeated vein injection of the extract can't cause evident inflammatory reaction to local veins.

In allergic test of cavia cobaya, 6 healthy cavia cobayas are separated to 2 groups in average. 0.5 ml the extract solution of which concentration is 0.5 g/ml is injected into belly cavity of all cavia cobayas respectively at the 1th, 3th and 5th day. Cavia cobayas of the first group is injected with 1ml the extract solution at 15th day. Cavia cobayas of the second group is injected with 1ml the extract solution at 22th day. To observe if there are phenomenons of piloerection (vertical hair), dyspnea, sneezing, dry vomiting, coughing, gong sound, twitching, atrophy, death and etc in 15 minutes after injection. Result: after all injections are completed, all the phenomenons never appear. It shows that the extract can't cause allergic response to cavia cobaya.

In haemolyticus test of rabbit, there isn't hemolysis or erythroagglutination. It shows that the extract can't cause hemolysis.

So the extract can be used as raw material to prepare many kinds of preparation. The present invention is also related to a kind of pharmaceutical combination containing the extract as active component. The pharmaceutical combination is tablets, capsule, drug granules, film agent, patch, coating, suppository, pilula, pulvis, ointment, mixture(fluid by mouth), syrups, tinct, eye preparation, nasal preparation, injection, asepsis powder for injection, concentrated solution for injection or their sustained-release preparation or controlled release preparation. All the preparation is routine pharmaceutical preparation, so the above preparations can be obtained with routine pharmaceutical technology in this field.

The extract or pharmaceutical combination which can prevent or treat thrombotic diseases can be used to prepare drugs for preventing or treating thrombotic diseases. For example the drug can prevent and treat hyperlipemia, artherosclerosis, myocardial infarction, angina, atherosclerotic infarct of brain, cerebral embolism, thrombosis of vein, lung embolism, pulmonary infarction, thromboangiitis obliterans, arteriosclerosis occlusion, disseminated inravascular coagulation (DIC), surgery complicated by thrombogenesis and thromboembolism, infarction of liver, infarction of kidney, infarction of cholecyst, infarction of mesentery,limbs gangrene,diabetes peripheral neuropathy, retina blood embolism or sudden deafness and so on.

The prominent advantage of the extract which can prevent or treat thrombotic diseases is: molecular weight of its all components is less than 5,800 Dalton, so it never or hardly initiates immunereaction. Because its relative molecular weight is low. Comparing with present other extract of leech and/or earthworm, amount and diameter of insolubility microparticles in the extract of the present invention are less and smaller, so the security of the extract of the present invention is improved evidently. Comparing with present other extract of leech and/or earthworm, activity or therapeutical effect of the extract of the present invention is equivalent or better. The extract of the present invention can be used without dermal test, this can shorten time of saving patients and lessen suffering of patients.

These and other objectives, features, and advantages of the present invention will become apparent from the following detailed description, the accompanying drawings, and the appended claims.

Brief Description of the Drawings

Fig. 1 is a high performance liquid chromatogram detection drawing of insulin human (5,800 Dalton).

Fig. 2 is a high performance liquid chromatogram detection drawing of extract of leech and/or earthworm in the present invention.

Fig. 3 is a mass spectrometric detection drawing of extract of leech in the present invention, wherein the base material is CCA, and the detection is positive ion.

Fig. 4 is a mass spectrometric detection drawing of extract of earthworm in the present invention, the base material is DHB, and the detection is negative ion.

Detailed Description of the Preferred Embodiment

Embodiment 1:

Equivalent quantity of dry leech and earthworm are soaked in normal saline or water for injection so that their surface can be spreaded adequately, then they are washed with normal saline or water for injection repeatedly and put into normal saline or water for injection of 2-4 times volume at 0-4 degree to be steeped for 24 hours and filtered, and then filtrate and dregs are preserved for use.

The dregs are pounded to pieces with tissue gravity mill. The filtrate is mixed with collid mill, and then the mixture is grinded to homogenate of which diameter is less than 0.5 micron. The homogenate is frozen at -15 °C for 15 -30 hours and melted at 0-4°C. Then above process is repeated at least 2 times.

The freeze thawing liquid is centrifugated with centrifuge and supernate is obtained. Then the supernate is filtered with ultrafiltration column which can intercept materials with molecular weight of more than 50,000 Dalton. Obtained filtrate is filtered with ultrafiltration column which can intercept materials with molecular weight of more than 10,000 Dalton again.

The above finally obtained filtrate is filtered with ultrafiltration column which can intercept materials with molecular weight of more than 6,000 Dalton. The filtrate is thermocompressed at 105-136°C for 10-45minutes and filtered with ultrafiltration column which can intercept materials with molecular weight of more than 6,000 Dalton again. Then extract liquid is obtained.

The extract liquid is dewatered and dried to solid if it is necessary.

There are polypeptide, amino acids and hypoxanthine in the extract through determination as above method. There are not materials with molecular weight of more than 5,800 Dalton in the extract, so molecular weight of all obtained materials in the extract is less than 5,800 Dalton. Chromatographic detection result is shown in figure 1 and 2.

All data of peaks in figure 1 and 2 is shown in table 1 and 2.

Table 1 detection result of insulin human in figure 1

| Number of peak | Retention time (min) | Type | Width (min) | Area (mAU) | Percent of area (%) |
|---|---|---|---|---|---|
| 1 | 12.027 | BB | 0.2851 | 3618.27271 | 100 |
| Total area: 3618.27271 | | | | | |

Table 2 detection result of extract in the present invention in figure 2

| Number of peak | Retention time (min) | Type | Width (min) | Area (mAU) | Percent of area (%) |
|---|---|---|---|---|---|
| 1 | 12.027 | | 0.0000 | 0.00000 | 0.0000 |
| 2 | 13.592 | BV | 0.8089 | 2312.62329 | 10.6292 |
| 3 | 14.847 | VV | 0.6827 | 4560.51563 | 20.9610 |
| 4 | 15.491 | VV | 0.2442 | 4643.37012 | 21.3418 |
| 5 | 16.095 | VV | 0.2940 | 2566.93359 | 11.7981 |
| 6 | 16.391 | VV | 0.3904 | 4863.65332 | 22.3542 |
| 7 | 18.802 | VV | 0.6900 | 1752.47546 | 8.0547 |
| 8 | 19.298 | VP | 0.5804 | 1057.62390 | 4.8610 |
| Total area: 2.17572e4 | | | | | |

Embodiment 2:

Equivalent quantity of dry or fresh leech and earthworm are soaked in normal saline or water for injection respectively so that their surface can be spreaded adequately, then they are washed with normal saline or water for injection repeatedly and put into normal saline or water for injection of 2-4 times volume at 0-4 degree to be steeped for 24 hours and filtered, and then filtrate and dregs are preserved for use.

The dregs are pounded to pieces with tissue gravity mill. The filtrate is mixed with collid mill, and then the mixture is grinded to homogenate of which diameter is less than 0.5 micron. The homogenate is frozen at -15 °C for 15-30 hours and melted at 0-4°C. Then above process is repeated at least 2 times.

The freeze thawing liquid is centrifugated with centrifuge and supernate is obtained. Then the supernate is filtered with ultrafiltration column which can intercept materials with molecular weight of more than 50,000 Dalton. Obtained filtrate is filtered with ultrafiltration column which can intercept materials with molecular weight of more than 10,000 Dalton again, and filtrate is obtained.

The filtrate is adjusted to pH 6.0-7.0 with phosphate buffer , then passes CM-cross-link glucan cation exchange column and eluted with buffer of 5 times volume at the same time. The eluent is collected and adjusted to pH 6.0-7.0 again, then passes DEAE-cross-link glucan anion exchange column and eluted with buffer. The eluent is abandoned, and then eluted with mixture of buffer of pH 6.0-7.0 and 0.2-0.5 mol/L sodium chloride. The eluent is collected and is filtered with ultrafiltration column which can intercept materials with molecular weight of more than 6,000 Dalton and desalted, then extract liquid is obtained. Extract liquid of leech and extract liquid of earthworm is obtained respectively, then they are mixed, and extract of leech and earthworm is obtained.

The extract liquid is dewatered and dried to solid if it is necessary.

There are polypeptide, amino acids and hypoxanthine in the extract through determination as above method. There are not materials with molecular weight of more than 5,800 Dalton in the extract, so molecular weight of all obtained materials in the extract is less than 5,800 Dalton.

Embodiment 3:

Equivalent quantity of dry or fresh leech and earthworm are soaked in normal saline or water for injection respectively so that their surface can be spreaded adequately, then they are washed with normal saline or water for injection repeatedly and put into normal saline or water for injection of 2-4 times volume at 0-4 degree to be steeped for 24 hours and filtered, and then filtrate and dregs are preserved for use.

The dregs are pounded to pieces with tissue gravity mill. The filtrate is mixed with collid mill, and then the mixture is grinded to homogenate of which diameter is less than 0.5 micron. The homogenate is frozen at -15 °C for 15-30 hours and melted at 0-4°C. Then above process is repeated at least 2 times.

The freeze thawing liquid is centrifugated with centrifuge and supernate is obtained. Then the supernate is filtered with ultrafiltration column which can intercept materials with molecular weight of more than 50,000 Dalton. Obtained filtrate is filtered with ultrafiltration column which can intercept materials with molecular weight of more than 10,000 Dalton again.

The above finally obtained filtrate is filtered with ultrafiltration column which can intercept materials with molecular weight of more than 6,000 Dalton. The filtrate is thermocompressed at 105-136°C for 10-45minutes and filtered with ultrafiltration column which can intercept materials with molecular weight of more than 6,000 Dalton again. Then extract liquid is obtained. And extract of leech and extract of earthworm is obtained respectively.

Molecular weight of materials in extract of leech and extract of earthworm is less than 5,800Dalton through determination as above method of MS. The result is shown in figure 3 and 4.

Extract of leech and earthworm is obtained after the extract of leech and the extract of earthworm are mixed. The mixed extract is added with 4% mannitol and 0.5-1 % active carbon in order to eliminate pyrogen, then filtered with micropore film, subpackaged to ampoules, freeze-dried, sealed and determined, then freeze-dry injectable powder of the extract is obtained.

Embodiment 4:

Dry or fresh leech are soaked in normal saline or water for injection so that their surface can be spreaded adequately, then they are washed with normal saline or water for injection repeatedly and put into normal saline or water for injection of 2-4 times volume at 0-4 degree to be steeped for 24 hours and filtered, and then filtrate and dregs are preserved for use.

The dregs are pounded to pieces with tissue gravity mill. The filtrate is mixed with collid mill, and then the mixture is grinded to homogenate of which diameter is less than 0.5 micron. The homogenate is frozen at -15°C for 15-30 hours and melted at 0-4°C. Then above process is repeated at least 2 times.

The freeze thawing liquid is centrifugated with centrifuge and supernate is obtained. Then the supernate is filtered with ultrafiltration column which can intercept materials with molecular weight of more than 50,000 Dalton. Obtained filtrate is filtered with ultrafiltration column which can intercept materials with molecular weight of more than 10,000 Dalton again.

The above finally obtained filtrate is thermocompressed at 105 -136 °C for 10-45 minutes and filtered with ultrafiltration column which can intercept materials with molecular weight of more than 6,000 Dalton 2-3 times. Then extract liquid is obtained.

The extract liquid is added with 5.4% β-cyclodextrin, stirred uniformly, soaked at 45 °C for 30 minutes in order to enwrap fully and spray dried. And dry powder is obtained.

The dry powder is added with 1.5% silicon dioxide, 0.3% PVP and 1% hydroxymethylstarch. The mixture is mixed uniformly, granulated and stuffed into hard capsules. And every capsule contains 1g the dry powder.

Embodiment 5:

The extract is obtained with the method in embodiment 1.

The extract is added with 6.4% starch, mixed uniformly, dried and sieved. Powder for medicinal use is contained.

The powder is added with 0.1% silica powder and 0.1% magnesium stearate, mixed uniformly and stuffed into hard capsules.

Embodiment 6: influence to clotting time of mouse

20±2g male mice of Kunming species are separated to 3 groups at random, 16mice/group. The three group of mice are injected with 10ml/kg normal saline, 2.5 g/kg extract of ZL03148281.3 and 2.5 g/kg the extract of present invention (embodiment 1) in tail vein of mice respectively. To take blood in eye sockets of mice 15 minutes later and measure clotting time with method of capillary vessel. The result is shown in table 3.

Table 3 influence to clotting time of mouse ($\bar{x}\pm s$)

| Group | n | Dosage(g/kg) | Clotting time(s) | Extension of clotting time |
|---|---|---|---|---|
| Normal saline | 16 | 10.0 | 70.9±17.9 | - |
| Extract of ZL03148281.3 | 16 | 2.5 | 110.5±31.9* | 55.8 |
| Extract of present invention | 16 | 2.5 | 113.7±27.9*# | 60.4 |
| Ps: *P<0.01 comparing with normal saline group; #P>0.05 comparing with extract of ZL03148281.3 | | | | |

The result is shown in table 3: extract of ZL03148281.3 can prolong clotting time of mice markedly (Q=5.9637 comparing with normal saline group, P<0.01), extract of present invention also can prolong clotting time of mice markedly (Q=6.4456 comparing with normal saline group, P<0.01). These two extract can both prolong clotting time of mice markedly, but there isn't distinguished difference between them (Q=0.4819, P>0.05).

Embodiment 7: influence to platelet and platelet sticking rate of mouse

1. influence to platelet

220-270g male mice is separated to 3 groups, 10 rats/groups. The three groups of mice are injected with 5 ml/kg normal saline, 1.0 g/kg the extract of present invention and 1.0 g/kg extract of ZL03148281.3 in tail vein of mice respectively. To take blood in eye sockets of mice 15 minutes later and measure platelet with globulimeter. The result is shown in table 4.

2. influence to platelet sticking rate

220 - 270 g male mice is separated to 3 groups, 10 rats/groups. The three groups of mice are injected with 5ml/kg normal saline, 1.0 g/kg the extract of present invention and 1.0 g/kg extract of ZL03148281.3 in tail vein of mice respectively. To take blood in eye sockets of mice 15 minutes later and measure number of platelet. This is the number of platelet before sticking. 1.5 ml blood is put into tube in which there is 0.3 ml 3.8% natrium citricum solution. To mixed uniformly and take 1ml mixture into a silicon tube ring. The ring is put into vitro thrombogenesis-platelet adhesion reaction machine and run for 5 minutes at speed of 17 rpm. To take blood to measure number of platelet as number of platelet after sticking and calculate platelet sticking rate. The result is shown in table 4. Calculation method of platelet is as following:

sticking rate(%)=number of platelet before sticking−number of platelet after sticking/ number of platelet before sticking×100%

Table 4 influence to platelet and platelet sticking rate of rat

| group | n | Dosage(g/kg) | Platelet number ($10^3/mm^3$) | Reduction of platelet (%) | platelet sticking rate (%) |
|---|---|---|---|---|---|
| Normal saline | 10 | 5.0 | 171.1 ± 45.6 | - | - |
| Extract of ZL03148281.3 | 10 | 1.0 | 148.5 ± 22.1* | 12.2 | 37.5 ± 13.5 |
| Extract of present invention | 10 | 1.0 | 131.4 ± 17.2**# | 15.2 | 32.0 ± 12.9# |
| Ps: *P<0.05, ** P<0.01 comparing with normal saline group; #P>0.05 comparing with extract of ZL03148281.3 | | | | | |

The result is shown in table 4: extract of ZL03148281.3 can lower platelet number of normal mice markedly (Q=2.9260, P<0.05 comparing with normal saline group, P<0.01), extract of present invention also can lower platelet number of normal mice markedly (Q=5.1399, P<0.01 comparing with normal saline group, P<0.01). These two extract can both lower platelet number of normal mice, but there isn't distinguished difference between them (Q=2.2139, P>0.05). It is shown that extract of present invention and extract of ZL03148281.3 can both lower platelet number and platelet sticking rate and decrease formation of thrombus.

Embodiment 8: detection of visible extraneous material and insoluble microparticle

Extract of leech and earthworm (called extract of ZL03148281.3 below) is obtained from equivalent quantity of dry leech and dry earthworm according to method in ZL03148281.3.
1. detection of visible extraneous material: visible extraneous materials in extract of present invention (embodiment 1) and extract of ZL03148281.3 are detected respectively according to Chinese Pharmacopeia (2005 edition) preparation general rule injection item. The result is shown in table 5.

Table 5 detection result of visible extraneous material

| Item sample | Qualified rate of lamp detection | Short and small fiber |
|---|---|---|
| extract of present invention | 92.7% | 3.9% |
| extract of ZL03148281.3 | 63.7% | 28.5% |

The result shows that there is remarkable difference between extract of present invention and extract of ZL03148281.3 in visible extraneous material. Qualified rate of lamp detection and short and small fiber of extract of present invention are remarkable better than these of extract of ZL03148281.3. So extract of present invention is safer.
2. detection of insoluble microparticle: insoluble microparticle in extract of present invention (embodiment 1) and extract of ZL03148281.3 are detected respectively according to Chinese Pharmacopeia (2005 edition) preparation general rule injection item. The result is shown in table 6.

Table 6 detection result of insoluble microparticle

| Item sample | Microparticle of more than 10um (average value/ramus) | Microparticle of more than 25um (average value/ramus) |
|---|---|---|
| extract of present invention | 196/ramus | 65/ramus |
| extract of ZL03148281.3 | 250/ramus | 110/ramus |

The result shows that there is remarkable difference between extract of present invention and extract of ZL03148281.3 in insoluble microparticle. Insoluble microparticle number of extract of present invention is remarkable less than that of extract of ZL03148281.3. So extract of present invention is safer.
Above results shows that: there isn't evident difference in pharmaceutical action between extract of present invention and extract of ZL03148281.3, and the former is better than the latter in some indexes. In other words, extract of present invention is much safer with no less activity than the extract of ZL03148281.3.
One skilled in the art will understand that the embodiment of the present invention as shown in the drawings and described above is exemplary only and not intended to be limiting.

It will thus be seen that the objects of the present invention have been fully and effectively accomplished. Its embodiments have been shown and described for the purposes of illustrating the functional and structural principles of the present invention and is subject to change without departure from such principles. Therefore, this invention includes all modifications encompassed within the scope of the following claims.

## Claims

1. An extract of leech and earthworm for use in the prevention or treatment of thrombotic diseases, which is extracted from leech *Whitmania pigra Whitman, Hirudo nipponica Whitman or Whitnania ac-ranulata Whitman* and earthworm *Pheretima aspergillum, Pheretima vulgaris Chen, Pheretima guillelmi or Pheretinia pectinifera,* and which is **characterized by** a molecular weight of less than 5,800 Dalton.

2. An extract of leech and earthworm for use according to claim 1, wherein said extract is obtained by using ultrafiltration membrane or ultrafiltration column which can intercept materials with molecular weight of more than 6,000 Dalton.

3. An extract of leech and earthworm for use according to claim 1 or 2, wherein said extract is made by the following steps:

   (1) cleaning leech and earthworm with water for injection or normal saline, steeping at low temperature, filtering, and obtaining filtrate and dregs;
   (2) smashing said dregs, and then mixing them with said filtrate forming homogenate, freezing and melting said homogenate repeatedly, then centrifuging, and obtaining the supernate;
   (3) ultrafiltrating said supernate obtained at step (2) with ultrafiltration membrane or ultrafiltration column intercepting molecular weight of between 10,000-100,000 Dalton, and obtaining the filtrate; and
   (4) ultrafiltrating said filtrate obtained at step (3) with ultrafiltration membrane or ultrafiltration column which can intercept molecular weight of more than 6,000 Dalton and obtaining the filtrate; and

   optionally, said filtrate is ultrafiltrated repeatedly with ultrafiltration membrane or ultrafiltration column which can intercept molecular weight of more than 6,000 Dalton; and, optionally, the filtrate is thermocompressed before being filtered, for example, said filtrate is thermocompressed at 105-136 °C for 10-45 minutes.

4. An extract of leech and earthworm for use according to claim 1 or 2, wherein said extract is made by the following steps:

   (1) cleaning leech and earthworm with water for injection or normal saline, smashing to form homogenate, freezing and melting said homogenate repeatedly, then centrifuging, and obtaining the supernate;
   (2) ultrafiltrating said supernate obtained at step (1) with ultrafiltration membrane or ultrafiltration column which can intercept molecular weight of between 10,000-100,000 Dalton, and obtaining the filtrate;
   (3) processing said filtrate obtained at step (2) with ion-exchange chromatography; and
   (4) desalting said filtrate obtained at step (3), then ultrafiltrating with ultrafiltration membrane or ultrafiltration column which can intercept molecular weight of more than 6,000 Dalton, or ultrafiltrating said filtrate obtained at step (3) with ultrafiltration membrane or ultrafiltration column which can intercept molecular weight of more than 6,000 Dalton, and then desalting; and

   optionally, said filtrate is ultrafiltrated repeatedly with ultrafiltration membrane or ultrafiltration column which can intercept molecular weight of more than 6,000 Dalton.

5. The extract of leech and earthworm for use according to claim 3 or 4, wherein said extract contains polypeptides, amino acids and hypoxanthine, and wherein said leech and earthworm to be cleaned in step (1) is dry and/or fresh, and said leech and earthworm are extracted together or separately, and wherein said extract is obtained in liquid form or made solid by dewatering and drying.

6. A method of obtaining the extract as recited in any one of claims 1 to 5, comprising the following steps:

   (1) cleaning leech and earthworm with water for injection or normal saline, steeping at low temperature, filtering, and obtaining filtrate and dregs;
   (2) smashing said dregs, and then mixing them with said filtrate obtained at step (1) forming homogenate, freezing and melting said homogenate repeatedly, then centrifuging, and obtaining the supernate;
   (3) ultrafiltrating said supernate obtained at step (2) with ultrafiltration membrane or ultrafiltration column intercepting molecular weight of between 10,000-100,000 Dalton, and obtaining the filtrate; and
(4) ultrafiltrating with ultrafiltration membrane or ultrafiltration column which can intercept molecular weight of more than 6,000 Dalton and obtaining the filtrate; and

optionally, said filtrate is ultrafiltrated repeatedly with ultrafiltration membrane or ultrafiltration column which can intercept molecular weight of more than 6,000 Dalton; and optionally, the filtrate is thermocompressed before being filtered; for example, said filtrate is thermocompressed at 105-136 °C for 10-45minutes.

**7.** The extract of leech and earthworm for use according to any one of claims 1 to 5, wherein said extract is an active component of a pharmaceutical combination.

**8.** The extract of leech and earthworm for use according to claim 8, wherein said pharmaceutical combination is in the form of tablets, capsule, drug granules, film agent, patch, coating, suppository, pilula, pulvis, ointment, mixture (fluid by mouth), syrups, tinct, eye preparation, nasal preparation, injection, asepsis powder for injection, concentrated solution for injection or sustained-release preparation or controlled release preparation.

**9.** The extract of leech and earthworm for use according to any one of claims 1 to 5 or claims 8 to 9, wherein said use is made in the prevention or treatment of thrombotic diseases such as hyperlipemia, artherosclerosis, myocardial infarction, angina, atherosclerotic infarct of brain, cerebral embolism, thrombosis of vein, lung embolism, pulmonary infarction, thromboangiitis obliterans, arteriosclerosis occlusion, disseminated intravascular coagulation (DIC), surgery complicated by thrombogenesis and thromboembolism, infarction of liver, infarction of kidney, infarction of cholecyst, infarction of mesentery, limbs gangrene, diabetes peripheral neuropathy, retina blood embolism or sudden deafness.

## Patentansprüche

**1.** Verwendung eines Blutegel- und Regenwurm-Extrakts in der Prävention oder Behandlung von thrombotischen Erkrankungen, der extrahiert wird aus Blutegeln *Whitmania pigra Whitman, Hirudo nipponica Whitman* oder *Whitmania ac-ranulata Whitman* und Regenwürmern *Pheretima aspergillum, Pheretima vulgaris Chen, Pheretima guillelmi* oder *Pheretima pectinifera,* und der sich durch ein Molekulargewicht von weniger als 5800 Dalton auszeichnet.

**2.** Verwendung des Blutegel- und Regenwurm-Extrakts nach Anspruch 1, wobei der Extrakt mittels einer Ultrafiltrations-Membran oder Ultrafiltrations-Säule erhalten wird, die Stoffe mit einem Molekulargewicht größer als 6000 Dalton abscheiden kann.

**3.** Verwendung des Blutegel- und Regenwurm-Extrakts nach Anspruch 1, wobei der Extrakt durch folgende Schritte hergestellt wird:

(1) Säubern der Blutegel und Regenwürmer mit Wasser für Injektionszwecke oder gewöhnlicher Saline, Einweichen bei geringer Temperatur und Filtrieren, wobei ein Filtrat und ein Bodensatz erhalten wird;
(2) Zerkleinern des Bodensatzes und Mischen mit dem Filtrat unter Bildung eines Homogenisats, wiederholtes Gefrieren und Auftauen des Homogenisats und anschließendes Zentrifugieren, wobei ein Überstand erhalten wird;
(3) Ultrafiltrieren des in Schritt (2) erhaltenen Überstands mittels einer Ultrafiltrations-Membran oder Ultrafiltrations-Säule, um Molekulargewichte zwischen 10000 und 100000 Dalton abzuscheiden, wobei ein Filtrat erhalten wird, und
(4) Ultrafiltrieren des in Schritt (3) erhaltenen Filtrats mittels einer Ultrafiltrations-Membran oder Ultrafiltrations-Säule, die geeignet ist, um Molekulargewichte größer als 6000 Dalton abzuscheiden, wobei das Filtrat erhalten wird; und

gegebenenfalls, wiederholtes Ultrafiltrieren des Filtrats mittels einer Ultrafiltrations-Membran oder Ultrafiltrations-Säule, die geeignet ist, um Molekulargewichte größer als 6000 Dalton abzuscheiden; und
gegebenenfalls, Thermokompressieren des Filtrats vor dem Filtrieren, beispielsweise bei 105-136 °C für 10-45 Minuten.

**4.** Verwendung des Blutegel- und Regenwurm-Extrakts nach Anspruch 1 oder 2, wobei der Extrakt durch folgende Schritte hergestellt wird:

(1) Säubern der Blutegel und Regenwürmer mit Wasser für Injektionszwecke oder gewöhnlicher Saline, Zerkleinern zur Bildung eines Homogenisats, wiederholtes Gefrieren und Auftauen des Homogenisats und anschließendes Zentrifugieren, wobei ein Überstand erhalten wird;
(2) Ultrafiltrieren des in Schritt (2) erhaltenen Überstands mittels einer Ultrafiltrations-Membran oder Ultrafiltrations-Säule, die geeignet ist, um Molekulargewichte zwischen 10000 und 100000 Dalton abzuscheiden, wobei ein Filtrat erhalten wird;
(3) Aufarbeiten des in Schritt (2) erhaltenen Filtrats mittels Ionenaustausch-Chromatographie; und
(4) Entsalzen des in Schritt (3) erhaltenen Filtrats, dann Ultrafiltrieren mittels einer Ultrafiltrations-Membran oder Ultrafiltrations-Säule, die geeignet ist, um Molekulargewichte größer als 6000 Dalton abzuscheiden, oder Ultrafiltrieren des in Schritt (3) erhaltenen Filtrats mittels einer Ultrafiltrations-Membran oder Ultrafiltrations-Säule, die geeignet ist, um Molekulargewichte größer als 6000 Dalton abzuscheiden, und dann Entsalzen; und

gegebenenfalls, wiederholtes Ultrafiltrieren des Filtrats mittels einer Ultrafiltrations-Membran oder Ultrafiltrations-Säule, die geeignet ist, um Molekulargewichte größer als 6000 Dalton abzuscheiden.

**5.** Verwendung des Blutegel- und Regenwurm-Extrakts nach Anspruch 3 oder 4, wobei der Extrakt Polypeptide, Aminosäuren und Hypoxanthine enthält, und wobei die in Schritt (1) zu säubernden Blutegel und Regenwürmer im getrockneten und/oder frischen Zustand sind, und Blutegel und Regenwurm zusammen oder getrennt extrahiert werden, und wobei der Extrakt in flüssiger Form erhalten wird oder, durch Entwässern und Trocknen, in Form eines Feststoffs.

**6.** Verfahren zur Herstellung eines Extrakts nach einem der Ansprüche 1 bis 5, umfassend die folgenden Schritte:

(1) Säubern der Blutegel und Regenwürmer mit Wasser für Injektionszwecke oder gewöhnlicher Saline, Einweichen bei geringer Temperatur und Filtrieren, wobei ein Filtrat und ein Bodensatz erhalten wird;
(2) Zerkleinern des Bodensatzes und Mischen mit dem in Schritt (1) erhaltenen Filtrat unter Bildung eines Homogenisats, wiederholtes Gefrieren und Auftauen des Homogenisats und anschließendes Zentrifugieren, wobei ein Überstand erhalten wird;
(3) Ultrafiltrieren des in Schritt (2) erhaltenen Überstands mittels einer Ultrafiltrations-Membran oder Ultrafiltrations-Säule, um Molekulargewichte zwischen 10000 und 100000 Dalton abzuscheiden, wobei ein Filtrat erhalten wird; und
(4) Ultrafiltrieren mittels einer Ultrafiltrations-Membran oder Ultrafiltrations-Säule, die geeignet ist, um Molekulargewichte größer als 6000 Dalton abzuscheiden, wobei das Filtrat erhalten wird; und

gegebenenfalls, wiederholtes Ultrafiltrieren des Filtrats mittels einer Ultrafiltrations-Membran oder Ultrafiltrations-Säule, die geeignet ist, um Molekulargewichte größer als 6000 Dalton abzuscheiden; und
gegebenenfalls, Thermokompressieren des Filtrats vor dem Filtrieren, beispielsweise bei 105-136 °C für 10-45 Minuten.

**7.** Verwendung des Blutegel- und Regenwurm-Extrakts nach einem der Ansprüche 1 bis 5, wobei der Extrakt eine aktive Komponente einer pharmazeutischen Zusammensetzung ist.

**8.** Verwendung des Blutegel- und Regenwurm-Extrakts nach Anspruch 8, wobei die pharmazeutischen Zusammensetzung in Form einer Tablette, einer Kapsel, eines Arzneistoff Granulats, eines Filmbildners, eines Pflasters, eines Überzugs, eines Suppositoriums, einer Pille, eines Pulvers, einer Salbe, eines Gemischs (welches im Mund flüssig wird), eines Sirups, einer Tinktur, einer ophthalmologische Zubereitung, einer nasale Zubereitung, einer Injektion, eines keimfreien Pulvers zur Injektion, einer konzentrierten Lösung zur Injektion oder eines Präparats zur dauerhaften Freisetzung oder gesteuerten Freisetzung, vorliegt.

**9.** Verwendung des Blutegel- und Regenwurm-Extrakts nach einem der Ansprüche 1 bis 5 oder 8 bis 9, wobei die Anwendung in der Prävention oder Behandlung von thrombotischen Erkrankungen erfolgt, wie Hyperlipidämie, Artherosklerose, Myokardinfarkt, Angina, artherosklerotischer Hirninfarkt, zerebrale Embolie, Venenthrombose, Lungenembolie, Lungeninfarkt, Endangiitis obliterans, Arteriosklerose, disseminierte intravasale Gerinnung (DIC), chirurgische Komplikationen auf Grund von Thrombogenese und Thromboembolien, Leberinfarkt, Niereninfarkt, Galleninfarkt, Infarkt des Gekröses, Gangrän der Gliedmaßen, diabetische periphere Neurophatie, Embolie der Retina-Blutgefäße oder Gehörsturz.

## Revendications

1. Extrait de sangsue et ver de terre pour Utilisation dans la prevention ou dans le traitement des maladies thrombotiques, qui est extraite à partir de la sangsue Whitmania pigra Whitman, Hirudo nipponica Whitman ou Whitmania ac-ranulata Whitman et du ver de terre Pheretima aspergillum, Pheretima vulgaris Chen, Pheretima guillelmi) ou Pheretima pectinifera et qui est **caractérisé par** un poids moléculaire inférieur à 5800 daltons.

2. L'extrait de sangsue et ver de terre pour l'utilisation selon la revendication 1, dans lequel ledit extrait est obtenu avec une membrane d'ultrafiltration ou une colonne d'ultrafiltration qui peut intercepter des matériaux ayant un poids moléculaire de plus de 6000.

3. L'extrait de sangsue et ver de terre pour l'utilisation selon la revendication 1 ou 2, dans lequel l'extrait est réalisé par les étapes suivantes:

   (1) le nettoyage de la sangsue et du ver de terre avec de l'eau pour injection ou une solution saline normale, trempage à basse température, filtration, et obtention du filtrat et des dépôts
   (2) l'écrasement des dépôts, puis les mélangeant avec ledit filtrat en formant un homogenate, refroidissement et fusion dudit homogenat à plusieurs reprises, puis centrifugation, et l'obtention du liquide surnageant;
   (3) l'ultrafiltration du ledit liquide surnageant obtenu dans l'étape (2) avec une membrane d'ultrafiltration ou une colonne d'ultrafiltration interceptant un poids moléculaire compris entre 10.000-100.000 Dalton, et l'obtention d'un filtrat; et
   (4) l'ultrafiltration dudit filtrat obtenu dans l'étape (3) avec une membrane d'ultrafiltration ou une colonne d'ultrafiltration qui peut intercepter un poids moléculaire inférieur à 6000 daltons et l'obtention d'un filtrat; et

   si nécessaire, ledit filtrat est ultrafiltré à plusieurs reprises avec une membrane d'ultrafiltration ou une colonne d'ultrafiltration qui peut intercepter un poids moléculaire de plus de 6000 daltons, et si nécessaire, le filtrat est thermocompressé avant d'être filtré, par exemple, ledit filtrat est thermocompressé à 105-136 °C pendant 10-45 minutes.

4. L'extrait de sangsue et ver de terre pour l'utilisation selon la revendication 1 ou 2, dans lequel ledit extrait est réalisé par les étapes suivantes:

   (1) le nettoyage de la sangsue et du ver de terre avec de l'eau pour injection ou une solution saline normale, broyage pour former un homogénat, refroidissement et fusion dudit homogenat à plusieurs reprises, puis centrifugation, et l'obtention du liquide surnageant;
   (2) l'ultrafiltration dudit liquide surnageant obtenu à l'étape (1) avec une membrane d'ultrafiltration ou une colonne d'ultrafiltration qui peut intercepter un poids moléculaire compris entre 10.000-100.000 Dalton, et l'obtention d'un filtrat;
   (3) le traitement dudit filtrat obtenu à l'étape (2) par chromatographie d'échange d'ions, et
   (4) l'élimination des sels dudit filtrat obtenu à l'étape (3), puis ultrafiltration avec une membrane d'ultrafiltration ou une colonne d'ultrafiltration qui peut intercepter un poids moléculaire de plus de 6000 daltons, ou ultrafiltration dudit filtrat obtenu à l'étape (3) avec une membrane d'ultrafiltration ou une colonne d'ultrafiltration qui peut intercepter un poids moléculaire de plus de 6000 daltons, et ensuite élimination des sels; et

   si nécessaire, ledit filtrat est ultrafiltré à plusieurs reprises avec une membrane d'ultrafiltration ou une colonne d'ultrafiltration qui peut intercepter un poids moléculaire de plus de 6000 daltons.

5. L'extrait de sangsue et ver de terre pour l'utilisation selon la revendication 3 ou 4, dans lequel ledit extrait contient des polypeptides, des acides aminés et de l'hypoxanthine, dans lequel ledit sangsue et ver de terre à nettoyer dans l'étape 2 est sec et/ou frais, et ledit sangsue et ver de terre sont extraits en même temps ou séparement, et dans lequel ledit extrait est obtenu sous forme liquide ou solidifié par déshydratation et séchage.

6. Procédé d'obtention de l'extrait selon l'une des revendications 1 à 5, comprenant des étapes suivantes:

   (1) le nettoyage de sangsue et ver de terre avec de l'eau pour injection ou une solution saline normale, trempage à basse température, filtration, et l'obtention du filtrate et des depots
   (2) l'écrasement dudits dépôts, et ensuite les mélanger avec ledit filtrat obtenu à l'étape (1) en formant un homogénat, refroidissement et fusion dudit homogénat à plusieurs fois, puis centrifugation, et l'obtention du liquide surnageant;

(3) l'ultrafiltration dudit liquide surnageant obtenu à l'étape (2) avec une membrane d'ultrafiltration ou une colonne d'ultrafiltration interceptant un poids moléculaire compris entre 10.000-100.000 Dalton, et l'obtention d'un filtrate; et

si nécessaire, ledit filtrat est ultrafiltré à plusieurs fois avec une membrane d'ultrafiltration ou une colonne d'ultrafiltration qui peut intercepter un poids moléculaire de plus de 6000 daltons et si nécessaire, le filtrat est thermocompressé avant d'être filter, par exemple, ledit filtrat est thermocompressé à 105-136°C pendant 10-45 minutes.

**7.** L'extrait de sangsue et ver de terre pour l'utilisation selon l'une des revendications 1 à 5, dans lequel ledit extrait est un composant actif d'une combinaison pharmaceutique.

**8.** L'extrait de sangsue et ver de terre pour l'utilisation selon la revendication 8, dans lequel la combinaison pharmaceutique est sous forme de comprimés, de capsules, de granules de médicament, d'agent de film, d'un tissue du revêtement, des suppositoires, des pilules, des pulvis, d'une pommade, d'un mélange (liquide par voie orale), de sirops, des teintures, d'une préparation pour l'oeil, d'une préparation nasale, d'injection, de poudre d'asepsie pour injection, d'une solution concentrée injectable ou d'une préparation à libération prolongée ou d'une préparation à libération contrôlée.

**9.** L'extrait de sangsue et ver de terre pour l'utilisation selon l'une des revendications 1 à 5 ou 8 à 9, dans lequel ladite utilisation est faite dans la prévention ou dans le traitement des maladies thrombotiques comme l'hyperlipidémie, l'athérosclérose, l'infarctus du myocarde, l'angine, l'infarctus athéroscléreuse du cerveau, l'embolie cérébrale, la thrombose de la veine, l'embolie pulmonaire, l'infarctus pulmonaire, la maladie de Buerger, l'artériosclérose d'occlusion, la coagulation intravasculaire disseminé (DIC), la chirurgie compliquée par thrombogénèse et de thromboembolie, l'infarctus du foie, l'infarctus du rein, l'infarctus du cholecyst, l'infarctus mésentérique, les membres gangrène, le diabète de neuropathie périphérique, la rétine embolie artérielle ou de surdité brusque.

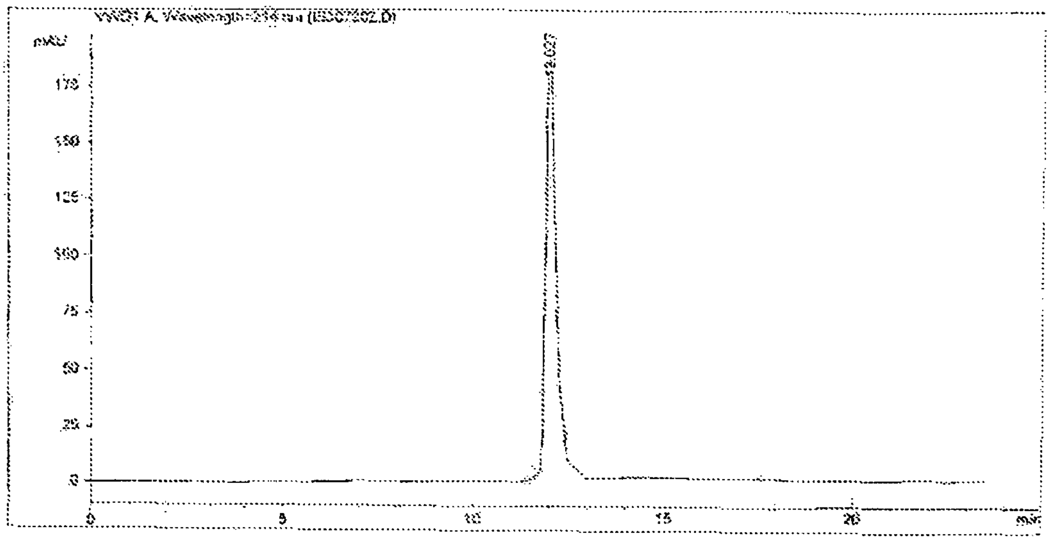


Area Percent Report

| Peak # | RetTime [min] | Type | Width [min] | Area mAU *s | Area % | Name |
|---|---|---|---|---|---|---|
| 1 | 12.027 | BB | 0.2851 | 3618.27271 | 100.0000 | Insulin |

Totals : 3618.37271

Results obtained with enhanced integrator!

*** End of Report ***

Instrument 1 8/15/03 8:03:30 AM CHENLIHONG

Page 1 of 1

Fig. 1

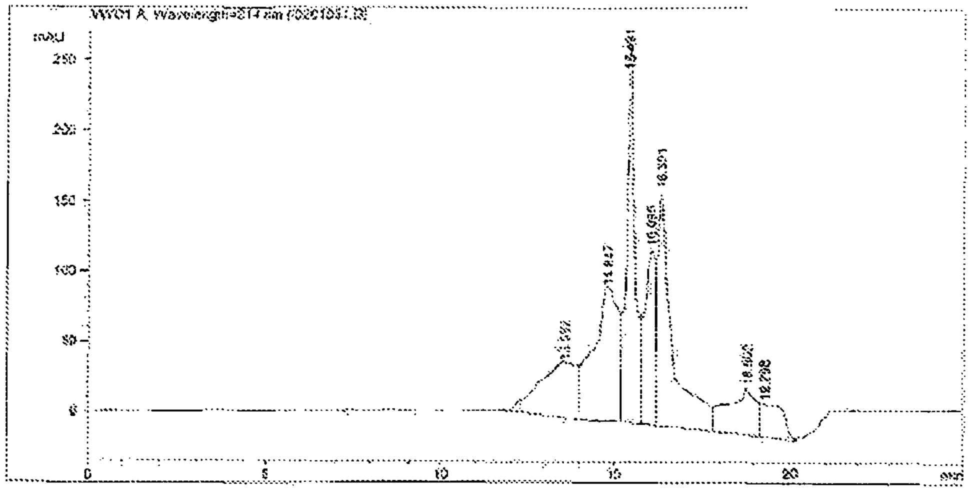


Area Percent Report

| Peak # | RetTime [min] | Type | Width [min] | Area mAU *s | Area % | Name |
|---|---|---|---|---|---|---|
| 1 | 12.037 | | 0.0000 | 0.00000 | 0.0000 | Insulin |
| 2 | 13.992 | BV | 0.8089 | 2312.62329 | 10.6292 | ? |
| 3 | 14.847 | VV | 0.6827 | 4560.51563 | 20.9610 | ? |
| 4 | 15.491 | VV | 0.2442 | 4643.37012 | 21.3418 | ? |
| 5 | 16.095 | VV | 0.2940 | 2566.93359 | 11.7981 | ? |
| 6 | 16.391 | VV | 0.3904 | 4863.65332 | 22.3542 | ? |
| 7 | 18.802 | VV | 0.6908 | 1752.47546 | 8.0547 | ? |
| 8 | 19.298 | VP | 0.5804 | 1057.62390 | 4.8610 | ? |

Totals : 2.17572e4

Results obtained with enhanced integrator!
1 Warnings or Errors :

Warning : Calibrated compound(s) not found

*** End of Report ***

Instrument 1 8/15/03 8:34:15 AM CHENLIHONG

Page 1 of 1

Fig. 2

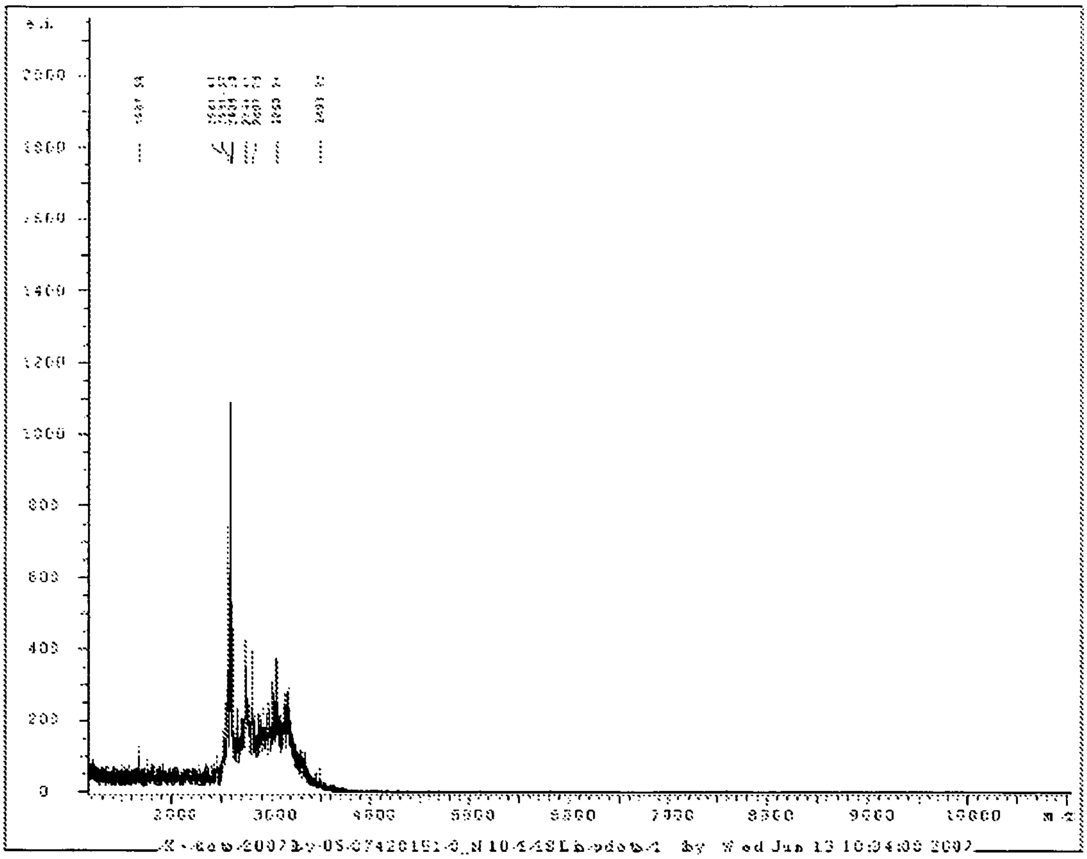
a.i.
2000
1800
1600
1400
1200
1000
800
600
400
200
0
2000
3000
4000
5000
6000
7000
8000
9000
10000
m/z

Fig. 3

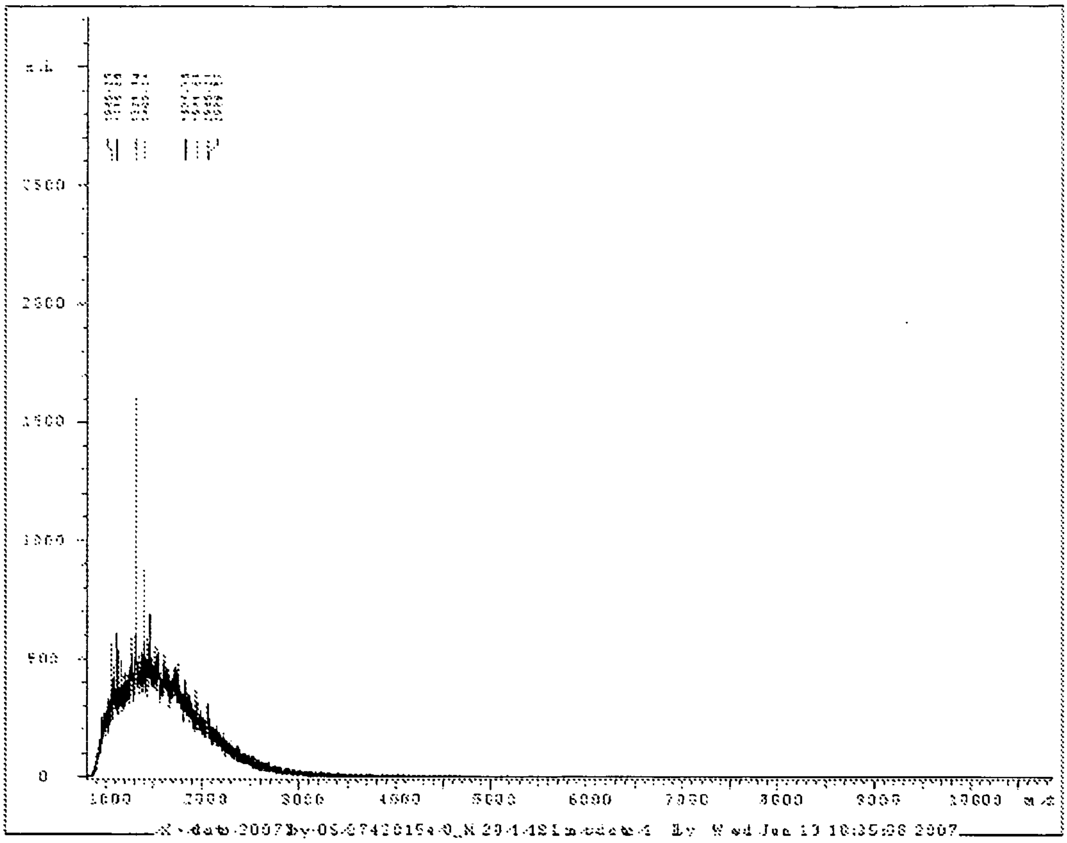

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 03148281
- CN 200410101538
- CN 200510000266
- US 5246715 A
- US 5861377 A
- WO 9717981 A
- CN 1192782 C
- CN 1651079 A
- CN 1660145 A


### Non-patent literature cited in the description


- **SEYMOUR JL et al.** *J. Biol. Chem.,* 1990, vol. 265 (17), 10143-10147
- **YANNES OSCAR et al.** *Mol. and Cell. Proteom.,* 2005, vol. 4 (10), 1602-1613
- Chinese pharmacopeia. 2005
- Chinese Pharmacopeia. 2005